# NEUE EUROPÄISCHE PATENTSCHRIFT

(11) **EP 0 599 241 B2**
(45) Veröffentlichungstag und Bekanntmachung des Hinweises auf die Entscheidung über den Einspruch: **24.01.2001**
(45) Hinweis auf die Patenterteilung: 13.08.1997
(21) Anmeldenummer: 93118754.6
(22) Anmeldetag: 22.11.1993
(51) Int. Cl.: C07C 45/43, C07C 47/54, C07C 47/55

(54) **Verfahren zur Herstellung aromatischer Aldehyde**
Process for the preparation of aromatic aldehydes
Procédé de préparation d'aldéhydes aromatiques

(30) Priorität: 26.11.1992 DE 4239737
(43) Veröffentlichungstag der Anmeldung: 01.06.1994
(73) Patentinhaber: Clariant GmbH, 65929 Frankfurt am Main (DE)
(72) Erfinder: Billeb, Gilbert, Dr., D-65779 Kelkheim/Ts (DE); Burg, Peter, D-65830 Kriftel/Taunus (DE)

(56) Entgegenhaltungen:
- JP-A- 60 218 349
- JP-A- 60 248 640
- NL-A- 7 215 589
- DATABASE WPI Week 8604, Derwent Publications Ltd., London, GB; AN 86-025603 & JP-A-60 248 640 (HODOGAYA CHEM IND KK) 9. Dezember 1985
- DATABASE WPI Week 8550, Derwent Publications Ltd., London, GB; AN 85-313611 & JP-A-60 218 349 (HODOGAYA CHEM IND KK) 1. November 1985
- CHEM. ABSTR., Bd. 84, 1976, Zusammenfassung Nr. 135232g & Pr. Inst. Przem. Org., 1974 (Pub. 1975) (6), 1-9
- ULLMANN, Enzyklopädie der technischen Chemie, 4. Aufl., Bd. A3, S. 465, 466, 1985
- CHEM. ABSTR., Bd. 104, 1986, Zusammenfassung Nr. 168138r & JP-A-60248640
- CHEM. ABSTR., Bd. 87, 1977, Zusammenfassung Nr. 84685j & JP-A-77025733
- CHEM. ABSTR., Bd. 86, 1977, Zusammenfassung Nr. 43407f & JP-A-7606129
- WINNACKER-KÜCHLER, Chemische Technologie, 4. Aufl., Bd. 6, S. 203

## Beschreibung

Aromatische Aldehyde sind wichtige Ausgangsprodukte zur Herstellung zahlreicher Verbindungen beispielsweise auf dem Gebiet der Pflanzenschutzmittel und Pharmazeutika.

Aromatische Aldehyde werden im allgemeinen durch Verseifung der entsprechenden dichlormethylsubstituierten Aromaten mit Wasser in Gegenwart von Säuren oder Metallsalzen gemäß der Reaktionsgleichung 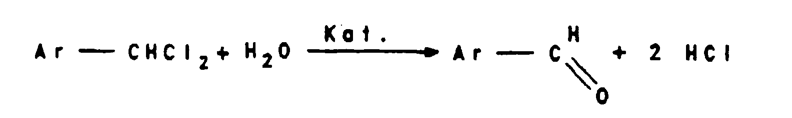 hergestellt (vgl. Houben-Weyl, VII, S. 211 ff.).

Diese Reaktion wird meist so durchgeführt, daß man den dichlormethylsubstituierten Aromat und den Katalysator bei erhöhter Temperatur vorlegt und Wasser zudosiert. Nach beendeter Reaktion wird entweder direkt destilliert, wobei der Katalysator in den Destillationsrückstand gerät, oder es wird wäßrig aufgearbeitet, wobei der Katalysator in das Abwasser gelangt.

Eine Ausnahme bildet ein Verfahren (BE 767 990), bei dem Eisen(III)-chlorid als Katalysator verwendet wird. Hierbei wird nach basischer Aufarbeitung das Eisen als Oxid bzw. Hydroxid gefällt und abgetrennt. Doch auch bei diesem Verfahren muß der Katalysator letztendlich entsorgt oder getrennt aufgearbeitet werden. Hinzu kommt, daß der Einsatz von Eisensalzen, die potentielle Friedel-Crafts-Katalysatoren sind, zu unerwünschten Nebenreaktionen (Friedel-Crafts-Reaktionen, Polymerisationen), die oft unkontrolliert verlaufen können, führen kann.

NL-A-7 215 589 beschreibt, daß die Hydrolyse mit wäßrigen Zinksalzlösungen durchgeführt werden kann, aber nur unter der Bedingung, daß vor der Reaktion mindestens ein Teil des Katalysators in fester Form zugesetzt wird. Dies ist aus verfahrenstechnischer Sicht von großem Nachteil.

Es bestand daher im Hinblick auf eine umweltverträgliche und kostengünstige Produktionsweise ein Bedürfnis nach einem verbesserten Verfahren zur Herstellung von gegebenenfalls substituierten Benzaldehyden, welches die genannten Nachteile nicht hat.

Es wurde nun überraschenderweise gefunden, daß man aromatische Aldehyde der allgemeinen Formel (1) 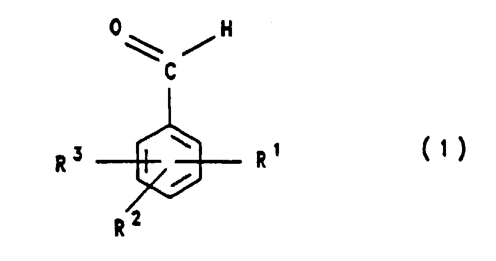 in welcher R¹, R², R³ unabhängig voneinander H, F, Cl oder Br, vorzugsweise H, F oder Cl darstellen, in vorteilhafter Weise kontinuierlich oder diskontinuierlich herstellen kann, indem man dichlormethylsubstituiertes Benzol der allgemeinen Formel (2) 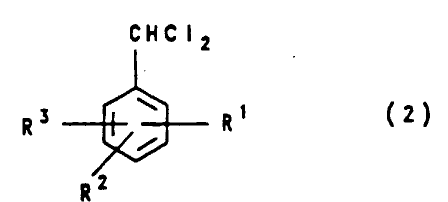 in welcher R¹, R², R³ die vorstehend genannten Bedeutungen haben, durch Zudosieren des gesamten Katalysators in Form einer 1 bis 50 %igen, vorzugsweise 1,1 bis 15 %igen wäßrigen Lösung eines oder mehrerer Zinksalze der allgemeinen Formel (3) in welcher X = F, Cl, Br, I, OH oder SO₄, vorzugsweise Cl, Br, OH oder SO₄ bedeutet und n in Abhängigkeit vom Anion X für die Zahl 1 oder 2 steht, bei Temperaturen von 70 bis 160°C, vorzugsweise 100 bis 140°C, hydrolysiert.

Im einzelnen sei zum erfindungsgemäßen Verfahren noch folgendes ausgeführt:

Das Benzalchlorid der genannten allgemeinen Formel (2) wird bei den vorstehend genannten Temperaturen vorgelegt. Dazu wird die wäßrige Katalysatorlösung zudosiert, wobei die Menge der Lösung durch den stöchiometrischen Verbrauch an Wasser vorgegeben ist und gegebenenfalls ein bis zu 100 %iger Überschuß zugesetzt werden kann Nach vollständiger Umsetzung des gegebenenfalls substituierten Benzalchlorides wird das Reaktionsgemisch mit Wasser extrahiert, wobei die Menge an Wasser so bemessen ist, daß eine mindestens 1 %ige Lösung des Zinksalzes entsteht. Vorzugsweise setzt man zur Extraktion in einem oder mehreren Anteilen so viel Wasser ein, wie bei der Reaktion stöchiometrisch verbraucht wurde. Die resultierende zinksalzhaltige wäßrige Phase wird zurückgeführt und wieder zur Verseifung eingesetzt. Die organische Phase ist anschließend frei von Zink und Salzsäure, was die weitere Aufarbeitung durch Destillation sehr vereinfacht und die Menge an Destillationsrückstand drastisch vermindert.

Bei der kontinuierlichen Verfahrensdurchführung wird die Verseifung der Verbindungen der genannten allgemeinen Formel (1) kontinuierlich in einer Rührkesselkaskade vorgenommen und das resultierende Reaktionsgemisch in der weiter oben beschriebenen Weise kontinuierlich mit Wasser behandelt und die resultirende zinksalzhaltige wäßrige Lösung kontinuierlich zurückgeführt. Die organische Phase wird in der weiter oben beschriebenen Weise durch Destillation aufgearbeitet.

Das Verfahren zeichnet sich insbesondere durch hohe Umweltverträglichkeit aus, da keine (schwermetallhaltigen Abwässer oder Destillationsrückstände entstehen. Der Katalysatorverbrauch ist gegenüber den herkömmlichen Verfahren drastisch reduziert, was zu erheblichen Kosteneinsparungen führt.

Daß beim erfindungsgemäßen Verfahren die wäßrigen Zinksalzlösungen sich so wirksam in dem gewünschten Sinn auswirken, war deshalb so überraschend, als diese Lösungen in der Literatur (J. Legocki et al., Pr. Inst. Przem. Org. 1974, 6, 1 - 9) als katalytisch unwirksam beschrieben wurden. Folgerichtig findet man in der Literatur kein Beispiel für und keine Hinweise auf eine Verseifung von dichlormethylsubstituierten Aromaten mit wäßrigen Zinksalz-Lösungen.

Durch die nachstehenden Beispiele wird das erfindungsgemäße Verfahren näher erläutert, ohne darauf beschränkt zu werden.

### Beispiel 1

195,3 g (1,0 Mol) 2-Chlorbenzalchlorid werden bei 120°C vorgelegt. Bei dieser Temperatur dosiert man innerhalb von 5 Stunden 20 ml (21,8 g, 1,09 Mol Wasser) einer 10 %igen wäßrigen Zinkchlorid-Lösung zu. Man rührt 30 min. bei 120°C nach. Nach Abkühlen auf Raumtemperatur extrahiert man das Reaktionsgemisch mit 1 x 10 ml und 2 x 5 ml Wasser. Die organische Phase ist anschließend chloridfrei und wird destillativ aufgearbeitet. Man erhält 136,0 g (97 % d. Th.) an 2-Chlorbenzaldehyd (Kp.: 90°C/16 Torr) in einer Reinheit von > 99,9 %. Die vereinigten wäßrigen Phasen (20 ml, 22,0 g) werden in Beispiel 2 als Katalysatorlösung eingesetzt.

### Beispiel 2

195,3 g (1,0 Mol) 2-Chlorbenzalchlorid werden analog Beispiel 1 verseift, wobei anstelle von frischer Zinkchlorid-Lösung das wäßrige Extrakt aus Beispiel 1 (20 ml) eingesetzt wird. Nach Aufarbeitung analog Beispiel 1 erhält man 136,3 g (97 % d.Th.) an 2-Chlorbenzaldehyd mit einer Reinheit von > 99,9 %. Man erhält 20 ml wäßriges Extrakt, welches wiederum in einer Verseifung eingesetzt wird. Insgesamt werden 5 Verseifungszyklen durchgeführt, wobei jeweils das wäßrige Extrakt des Vorversuchs eingesetzt wird:

| Zyklus | Ausbeute [g] | Ausbeute [%] |
|---|---|---|
| I | 136,3 | 97,0 |
| II | 135,5 | 96,4 |
| III | 137,0 | 97,5 |
| IV | 136,0 | 96,8 |
| V | 135,5 | 96,4 |

### Beispiel 3

Die Verseifung von 2-Chlorbenzalchlorid (Ansatz 195,3 g) wird analog Beispielen 1 und 2 mit 5 %iger wäßriger Zinkchlorid-Lösung durchgeführt, wobei folgende Resultate erhalten werden:

| Zyklus | Ausbeute [g] | Ausbeute [%] |
|---|---|---|
| I | 135,5 | 96,4 |
| II | 134,5 | 95,7 |
| III | 136,5 | 97,1 |
| IV | 137,0 | 97,5 |
| V | 135,5 | 96,4 |

### Beispiel 4

Die Verseifung von 4-Chlorbenzalchlorid (Ansatz 195,3 g) wird analog Beispielen 1 und 2 bei 100°C mit 10 %iger wäßriger Zinkchlorid-Lösung durchgeführt. Nach Destillation der organischen Phase bei 80°C/10 Torr erhält man folgende Resultate:

| Zyklus | Ausbeute [g] | Ausbeute [%] |
|---|---|---|
| I | 135,0 | 96,1 |
| II | 136,0 | 96,8 |
| III | 134,5 | 95,7 |
| IV | 135,2 | 96,2 |
| V | 137,0 | 97,5 |

### Beispiel 5

Die Verseifung von 2-Chlor-6-fluorbenzalchlorid (Ansatz 213,3 g) wird analog Beispielen 1 und 2 bei 140°C mit 10 %iger wäßriger Zinkchlord-Lösung durchgeführt. Nach Destillation der organischen Phase bei 90°C/10 Torr erhält man folgende Ergebnisse:

| Zyklus | Ausbeute [g] | Ausbeute [%] |
|---|---|---|
| I | 150,5 | 95,0 |
| II | 152,1 | 96,0 |
| III | 151,0 | 95,3 |
| IV | 152,9 | 96,5 |
| V | 153,5 | 96,9 |

### Beispiel 6

4-Fluorbenzalchlorid (Ansatz 178,8 g) wird analog Beispiel 4 bei 100°C mit 10 %iger wäßriger Zinkchlorid-Lösung verseift. Man erhält nach Destillation bei 70°C/10 Torr folgende Ergebnisse:

| Zyklus | Ausbeute [g] | Ausbeute [%] |
|---|---|---|
| I | 116,5 | 94,0 |
| II | 118,4 | 95,5 |
| III | 119,6 | 96,5 |
| IV | 117,8 | 95,0 |
| V | 120,0 | 96,8 |

### Beispiel 7

195,3 g (1,0 Mol) 2-Chlorbenzalchlorid werden analog Beispielen 1 und 2 mit 10 %iger wäßriger Zn(OH)₂-Lösung umgesetzt. Es werden im Vergleich zu Beispiel 2 keine signifikant unterschiedlichen Ergebnisse erhalten.

## Patentansprüche

1. Verfahren zur Herstellung aromatischer Aldehyde der allgemeinen Formel (1) 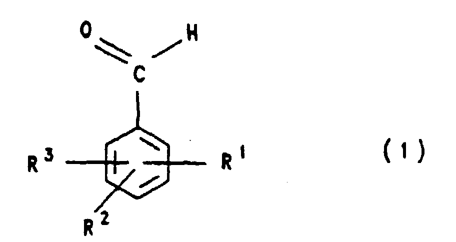 in welcher R¹, R², R³ unabhängig voneinander H, F, Cl oder Br darstellen, dadurch gekennzeichnet, daß man dichlormethylsubstituiertes Benzol der allgemeinen Formel (2) 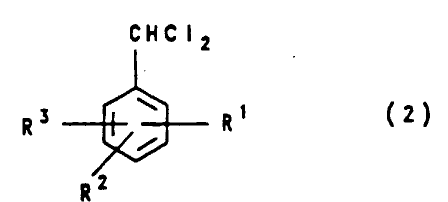 in welcher R¹, R², R³ die vorstehend genannten Bedeutungen haben, durch Zudosieren des gesamten Katalysators in Form einer 1 bis 50 %igen wäßrigen Lösung eines oder mehrerer Zinksalze der allgemeinen Formel (3)
ZnXₙ (3)
in welcher X = F, Cl, Br, l, OH oder SO₄ bedeutet, und n in Abhängigkeit vom Anion X für die Zahl 1 oder 2 steht, bei Temperaturen von 70 bis 160°C hydrolysiert.

2. Verfahren nach Anspruch 1, dadurch gekennzeichnet, daß R¹, R², R³ in den allgemeinen Formeln (1) und (2) H, F oder Cl bedeuten.

3. Verfahren nach mindestens einem der Ansprüche 1 und 2, dadurch gekennzeichnet, daß X in der allgemeinen Formel (3) Cl, Br, OH oder SO₄ bedeutet.

4. Verfahren nach mindestens einem der Ansprüche 1 bis 3, dadurch gekennzeichnet, daß man bei Temperaturen von 100 bis 140°C hydrolysiert.

5. Verfahren nach mindestens einem der Ansprüche 1 bis 4, dadurch gekennzeichnet, daß man mit einer 1,1 bis 15 %igen wäßrigen Lösung eines oder mehrerer Zinksalze der in Anspruch 1 genannten allgemeinen Formel (3) hydrolysiert.

6. Verfahren nach mindestens einem der Ansprüche 1 bis 6, dadurch gekennzeichnet, daß man das nach erfolgter Hydrolyse angefallene rohe Verseifungsgemisch mit Wasser extrahiert und die dabei erhaltene wäßrige zinksalzhaltige Lösung zurückführt und als wäßrige Katalysatorlösung wieder in die Verseifungsreaktion eingesetzt.

7. Verfahren nach mindestens einem der Ansprüche 1 bis 6, dadurch gekennzeichnet, daß man das nach erfolgter Hydrolyse angefallene rohe Verseifungsgemisch mit Wasser extrahiert, wobei die Menge an Wasser so bemessen ist, daß eine mindestens 1 %ige Lösung des Zinksalzes entsteht.

8. Verfahren nach mindestens einem der Ansprüche 1 bis 6, dadurch gekennzeichnet, daß man das nach erfolgter Hydrolyse angefallene rohe Verseifungsgemisch mit Wasser extrahiert, wobei man so viel Wasser einsetzt, wie bei der Reaktion stöchiometrisch verbraucht wurde.

9. Verfahren nach mindestens einem der Ansprüche 1 bis 8, dadurch gekennzeichnet, daß man die Verseifungsreaktion kontinuierlich in einer Rührkesselkaskade ausführt, das angefallene Reaktionsgemisch kontinuierlich mit Wasser behandelt und die hierbei resultierende zinksalzhaltige wäßrige Lösung kontinuierlich zurückführt.

## Claims

1. A process for preparing aromatic aldehydes of the formula (1) 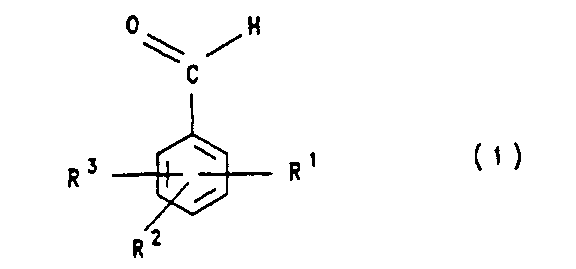 in which R¹, R², R³ independently of one another are H, F, Cl or Br, which comprises hydrolyzing a dichloromethyl-substituted benzene of the formula (2) 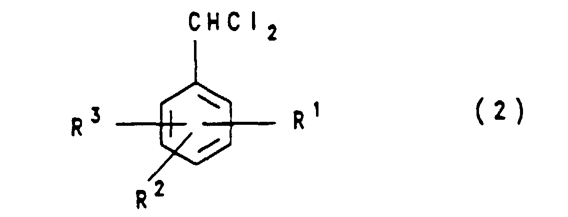 in which R¹, R², R³ have the abovementioned meanings by metering in all of the catalyst in the form of a from 1 to 50 % strength aqueous solution of one or more zinc salts of the formula (3)
ZnXₙ (3)
in which X is F, Cl, Br, I, OH or SO₄, and n is, depending on the anion X, the number 1 or 2, at temperatures of from 70 to 160°C.

2. The process as claimed in claim 1, wherein R¹, R², R³ in the formulae (1) and (2) are H, F or Cl.

3. The process as claimed in at least one of claims 1 and 2, wherein X in the formula (3) is Cl, Br, OH or SO₄.

4. The process as claimed in at least one of claims 1 to 3, wherein hydrolysis is carried out at temperatures from 100 to 140°C.

5. The process as claimed in at least one of claims 1 to 4, wherein hydrolysis is carried out with a from 1.1 to 15 % strength aqueous solution of one or more zinc salts of the formula (3) given in claim 1.

6. The process as claimed in at least one of claims 1 to 5, wherein the crude saponification mixture obtained after hydrolysis has been effected is extracted with water and the resulting aqueous zinc salt-containing solution is recirculated and reused as the aqueous catalyst solution in the saponification reaction.

7. The process as claimed in at least one of claims 1 to 6, wherein the crude saponification mixture obtained after hydrolysis has been effected is extracted with water, the amount of water being calculated so that an at least 1 % strength solution of the zinc salt is obtained.

8. The process as claimed in at least one of claims 1 to 6, wherein the crude saponification mixture obtained after hydrolysis has been effected is extracted with water, the amount of water used being the same as that which is stoichiometrically used during the reaction.

9. The process as claimed in at least one of claims 1 to 8, wherein the saponification reaction is carried out continuously in a cascade of stirred reactors, the reaction mixture obtained is treated continuously with water and the resulting zinc salt-containing aqueous solution is continuously recirculated.

## Revendications

1. Procédé de préparation d'aldéhydes aromatiques de formule générale (1) 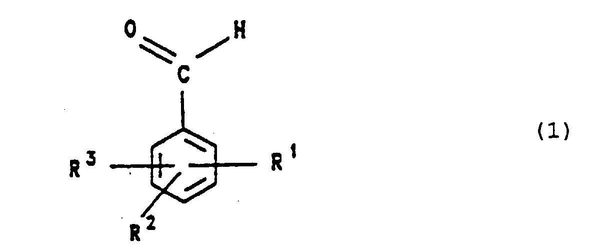 dans laquelle R¹, R², R³, indépendamment les uns des autres, représentent H, F, Cl ou Br, caractérisé en ce que l'on hydrolyse le benzène substitué par dichlorométhyle de formule générale (2) 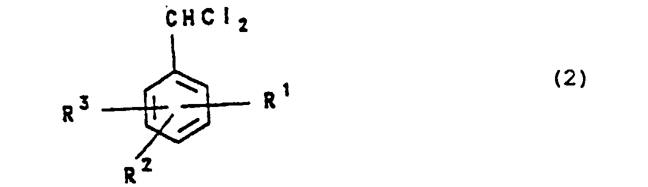 dans laquelle R¹, R², R³ ont les significations données précédemment, par addition progressive de tout le catalyseur sous forme d'une solution aqueuse à 1 à 50 % d'un ou plusieurs sels de zinc de formule générale (3)
ZnXₙ (3)
dans laquelle X = F, Cl, Br, I, OH ou SO₄ et n vaut 1 ou 2 en fonction de l'anion X, à des températures de 70 à 160°C.

2. Procédé selon la revendication 1, caractérisé en ce que R¹, R², R³ représentent dans les formules générales (1) et (2) H, F ou Cl.

3. Procédé selon au moins l'une des revendications 1 et 2, caractérisé en ce que X dans la formule générale (3) représente Cl, Br, OH ou SO₄.

4. Procédé selon au moins l'une des revendications 1 à 3, caractérisé en ce qu'on effectue l'hydrolyse à des températures de 100 à 140°C.

5. Procédé selon au moins l'une des revendications 1 à 4, caractérisé en ce qu'on effectue l'hydrolyse avec une solution aqueuse à 1,1 à 15 % d'un ou plusieurs sels de zinc de formule générale (3) citée dans la revendication 1.

6. Procédé selon au moins l'une des revendications 1 à 5, caractérisé en ce que l'on extrait avec de l'eau le mélange de saponification brut obtenu après l'hydrolyse et on recycle la solution aqueuse contenant du sel de zinc ainsi obtenue et on la réutilise dans la réaction de saponification comme solution aqueuse de catalyseur.

7. Procédé selon au moins l'une des revendications 1 à 6, caractérisé en ce que l'on extrait avec de l'eau le mélange de saponification brut obtenu après l'hydrolyse, la quantité d'eau étant calculée de façon telle qu'il se forme une solution du sel de zinc à 1% au moins.

8. Procédé selon au moins l'une des revendications 1 à 6, caractérisé en ce que l'on extrait avec de l'eau le mélange de saponification brut obtenu après l'hydrolyse, en utilisant une quantité d'eau correspondant à la consommation stoeechiométrique dans la réaction.

9. Procédé selon au moins l'une des revendications 1 à 8, caractérisé en ce que l'on met en oeuvre la réaction de saponification en continu dans une cascade de tubes à agitation, on traite en continu le mélange réactionnel formé par l'eau et on recycle en continu la solution aqueuse contenant du sel de zinc ainsi obtenue.
